# EUROPEAN PATENT APPLICATION

(11) **EP 3 235 391 A1**
(43) Date of publication of application: **25.10.2017**
(21) Application number: 16178897.1
(22) Date of filing: 11.07.2016
(51) Int. Cl.: A24F 47/00

(54) **ANTI-SEEPAGE ATOMIZER**

(30) Priority: 20.04.2016 CN 201610248617
(71) Applicant: Shenzhen Jieshibo Technology Co. Ltd., Shenzhen, Guangdong 518000 (CN)
(72) Inventor: WU, Jianyong, Shenzhen, Guangdong 518000 (CN)
(74) Representative: Sun, Yiming

(57) **Abstract**

The present invention discloses an anti-seepage atomizer, including an oil cup and a hollow atomizing core, where a hollow tube is disposed at a center of the oil cup in a manner of extending inward and downward from a top, an upper end of the atomizing core is movably inserted into the hollow tube in a sealed manner from a place below the hollow tube, a lower end of the atomizing core is movably disposed inside an atomizing core base in a sleeved manner, an inner wall of the oil cup, the hollow tube, an upper surface of the upper end of the atomizing core, and an upper surface of an atomizing core base form a sealed oil storage chamber, the middle of the atomizing core is provided with at least one oil hole in communication with an atomizing structure inside the atomizing core, if the atomizing core slides downward, the oil hole is hidden inside the atomizing core base so as to isolate the oil storage chamber from the atomizing structure in a sealed manner, and if the atomizing core slides upward, the oil hole is located inside the oil storage chamber so as to make the oil storage chamber in communication with the atomizing structure. An atomized liquid storage apparatus of the present invention has advantages of a simple structure and preferable sealing performance, and the anti-seepage atomizer of the present invention has an advantage that the atomized liquid would not be seeped during storage or transportation.

## Description

### Technical Field

The present invention relates to an atomizing apparatus, and in particular, to an anti-seepage atomizer where an atomized liquid is not evaporated or seeped during storage or transportation.

### Related Art

An atomizer is mainly used for inhaling fragrance or medicine. In an existing atomizer, a liquid storage device and an atomizing apparatus are in communication with each other by using absorption cotton or a fiber rope, and during use, a heating structure heats and evaporates the atomized liquid absorbed inside the absorption cotton or fiber rope, and the absorption cotton or fiber rope continuously guides the atomized liquid out from the liquid storage device; however, in this way, during storage or transportation, as the temperature increases or vibration occurs, the atomized liquid would also be evaporated or seeped from the atomizing apparatus through the absorption cotton or fiber rope.

### SUMMARY

To resolve the foregoing problem, an objective of the present invention is to provide an anti-seepage atomizer where an atomized liquid is not evaporated or seeped during storage or transportation.

The present invention is implemented by means of the following technical means. An anti-seepage atomizer includes an oil cup and a hollow atomizing core, where a hollow tube is disposed at a center of the oil cup in a manner of extending inward and downward from a top, an upper end of the atomizing core is movably inserted into the hollow tube in a sealed manner from a place below the hollow tube, a lower end of the atomizing core is movably disposed inside an atomizing core base in a sleeved manner, an inner wall of the oil cup, the hollow tube, an upper surface of the upper end of the atomizing core, and an upper surface of an atomizing core base form a sealed oil storage chamber, the middle of the atomizing core is provided with at least one oil hole in communication with an atomizing structure inside the atomizing core, if the atomizing core slides downward, the oil hole is hidden inside the atomizing core base so as to isolate the oil storage chamber from the atomizing structure in a sealed manner, and if the atomizing core slides upward, the oil hole is located inside the oil storage chamber so as to make the oil storage chamber in communication with the atomizing structure.

In a preferable manner, an elastic structure is disposed between the atomizing core and the atomizing core base, the atomizing core slides upward to elastically deform the elastic structure, and the atomizing core slides downward to elastically recover the elastic structure.

In a preferable manner, the atomizing structure is a spiral heating body, a long strip-shaped heating body, a sheet-shaped heating body, or a porous circular tube-shaped heating body that is disposed in a same direction as that of the hollow tube, and the spiral heating body, the long strip-shaped heating body, the sheet-shaped heating body, or the porous circular tube-shaped heating body is wrapped by an absorption fiber around.

In a preferable manner, the spiral heating body, the long strip-shaped heating body, the sheet-shaped heating body, or the porous circular tube-shaped heating body performs resistance heating or electromagnetic heating.

In a preferable manner, the atomizing structure is a hollow heating body, the atomized liquid is guided to a hollow body, the hollow heating body is provided with a micro hole, steam-fog generated by means of atomization when the hollow heating body performs heating is discharged through the micro hole, and the hollow heating body performs resistance heating or electromagnetic heating.

In a preferable manner, atomizing structure is a fiber rope disposed inside an atomizing core cavity, and a surface of the fiber rope is wound by an electrical heating wire.

In a preferable manner, an atomizer machine body is further included, a pressing structure is disposed inside the atomizer machine body, and when the oil cup is connected to the atomizer machine body, the pressing structure presses the atomizing core to enable the atomizing core to slide upward.

In a preferable manner, a battery is disposed inside the atomizer machine body, and an electrode electrically connected to the battery is disposed below the atomizing core.

In a preferable manner, the oil cup, the atomizing core, or the atomizing core base is provided with an identification code structure, and the atomizer machine body is provided with a code reading structure.

In a preferable manner, the identification code structure is a conductive contact point, a radio frequency tag, a two-dimensional code, a graphical code, or an identification chip that is arranged according to a specific structure.

In a preferable manner, a control main board is disposed inside the atomizer machine body, a surface of the atomizer machine body is further provided with a display screen electrically connected to the control main board, and a wireless transmit module electrically connected to the control main board is further disposed inside the atomizer machine body.

In the present invention, an inner wall of an oil cup, a hollow tube, an upper surface of an upper end of an atomizing core, and an upper surface of an atomizing core base form a sealed oil storage chamber; when use is needed, the atomizing core is enabled to slide upward, and then an oil hole is located inside the oil storage chamber to make the oil storage chamber in communication with an atomizing structure; and when use it not performed, the atomizing core slides downward, and then the oil hole is hidden inside the atomizing core base to isolate the oil storage chamber from the atomizing structure in a sealed manner. In the present anti-seepage atomizer, because when the anti-seepage atomizer is not used, the oil storage chamber is isolated from an atomizing core cavity in a sealed manner, an atomized liquid inside the atomizing structure would not be seeped from the atomizing core during storage or transportation. The present anti-seepage atomizer may be applied to an electronic cigarette, medicine atomizing treatment, and the like.

### BRIEF DESCRIPTION OF THE DRAWINGS

FIG. 1 is a schematic breakdown structure diagram of an embodiment of the present invention;
FIG. 2 is a sectional view during use of an atomizing apparatus of an embodiment of the present invention; and
FIG. 3 is a sectional view during use of an atomizing apparatus of another embodiment of the present invention.

### DETAILED DESCRIPTION

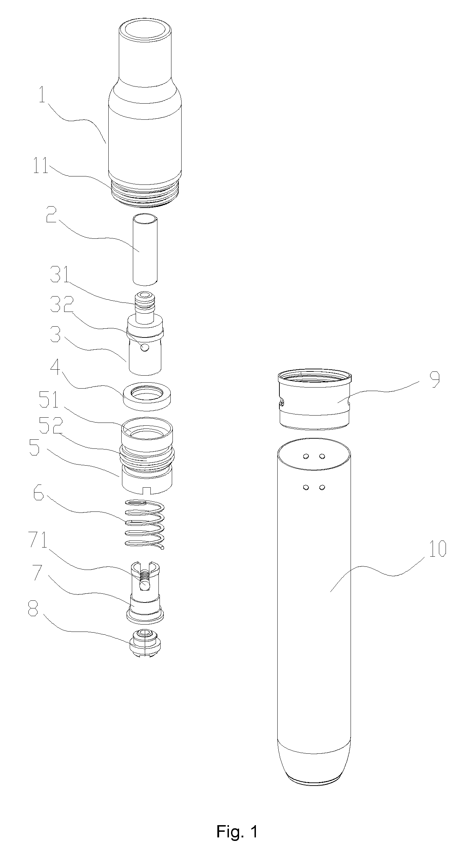

In the present embodiment, an anti-seepage atomizer, referring to FIG. 1 to FIG. 3, includes an oil cup 1 and a hollow atomizing core, where a hollow tube is disposed at a center of the oil cup in a manner of extending inward and downward from a top, an upper end of the atomizing core is movably inserted into the hollow tube in a sealed manner from a place below the hollow tube, a lower end of the atomizing core is movably disposed inside an atomizing core base 5 in a sleeved manner, an inner wall of the oil cup 1, the hollow tube, an upper surface of the upper end of the atomizing core, and an upper surface of an atomizing core base 5 form a sealed oil storage chamber, the middle of the atomizing core is provided with at least one oil hole 32 in communication with an atomizing structure inside the atomizing core, if the atomizing core slides downward, the oil hole 32 is hidden inside the atomizing core base 5 so as to isolate the oil storage chamber from the atomizing structure in a sealed manner, and if the atomizing core slides upward, the oil hole 32 is located inside the oil storage chamber so as to make the oil storage chamber in communication with the atomizing structure.

Because, in the present anti-seepage atomizer, the inner wall of the oil cup 1, the hollow tube, the upper surface of the upper end of the atomizing core, and the upper surface of the atomizing core base 5 form a sealed oil storage chamber; when use is needed, the atomizing core is enabled to slide upward, and then an oil hole 32 is located inside the oil storage chamber to make the oil storage chamber in communication with an atomizing structure; and when use it not performed, the atomizing core slides downward, and then the oil hole 32 is hidden inside the atomizing core base to isolate the oil storage chamber from the atomizing structure in a sealed manner. In the present anti-seepage atomizer, because when the anti-seepage atomizer is not used, the oil storage chamber is isolated from an atomizing core cavity in a sealed manner, an atomized liquid inside the atomizing structure would not be seeped from the atomizing core during storage or transportation. The present anti-seepage atomizer may be applied to an electronic cigarette, medicine atomizing treatment, and the like.

In an anti-seepage atomizer of an embodiment, on the basis of the preceding technical solution, specifically, the atomizing structure is a spiral heating body that is disposed in a same direction as that of the hollow tube (which is formed by winding an electrical heating wire and if electrical induction heating is adopted, is formed by winding a metal wire) and is wrapped by an absorption fiber or another liquid absorption material around; and alternatively, may be a long strip-shaped heating body, a sheet-shaped heating body, or a porous circular tube-shaped heating body and be further wrapped by an absorption fiber around.

In an anti-seepage atomizer of an embodiment, on the basis of the preceding technical solution, specifically, the atomizing structure is a hollow heating body, the atomized liquid is guided to a hollow body, the hollow heating body is provided with a micro hole, and steam-fog generated by means of atomization when the hollow heating body performs heating is discharged through the micro hole. The hollow heating body can perform resistance heating directly or may be a metal tube, of which a periphery is provided with an inductive coil to perform electromagnetic heating.

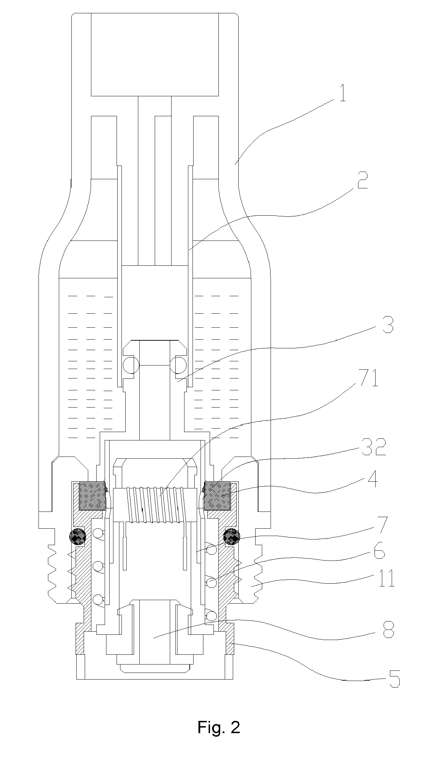

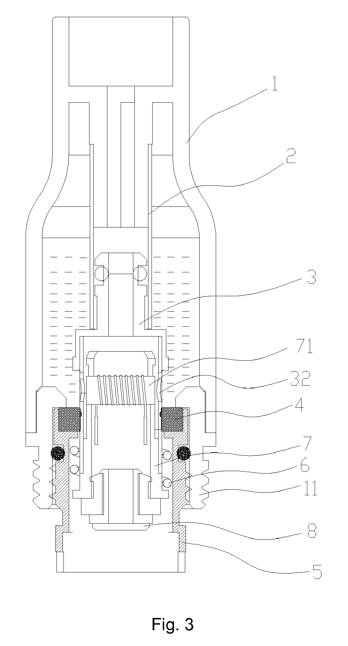

In an anti-seepage atomizer of an embodiment, referring to FIG. 1 to FIG. 3, on the basis of the preceding technical solution, specifically, a center of the oil cup 1 extends inward and downward and is provided with a steel tube 2 in sleeved manner to form a hollow tube for an atomized gas to flow out; the atomizing core includes an atomizing core jacket 3, an atomizing core cap 33 is fixed above the atomizing core jacket 3, and an upper end of the atomizing core cap 33 is provided with a small tube 31 capable of sliding inside the steel tube, the middle of the small tube 31 is provided with an ewe neck, and the ewe neck is provided with a sealing rubber ring in a sleeved manner; three oil holes 32 are disposed above the atomizing core jacket 3, an electrical heating wire rack 7 provided with a electrical heating wire 71 is inserted from a position below the atomizing core jacket 3, the electrical heating wire 71 winds a fiber rope, and a diameter of a lower bottom edge of the electrical heating wire rack 7 is greater than that of the atomizing core jacket 3; the atomizing core base 5 is hollow, and the middle thereof is provided with an inward convex ring 51, and a diameter of the convex ring 51 is between the diameter of the atomizing core jacket 3 and the diameter of the lower bottom edge of the electrical heating wire rack 7; the atomizing core penetrates through the atomizing core base 5; the atomizing core jacket 3 is provided with a spring 6 in a sleeved manner, the spring 6 is disposed between the convex ring 51 and the lower bottom edge of the electrical heating wire rack 7; a silicon gasket 4 pressing an upper surface of the convex ring 51 is embedded above the atomizing core base 5, and the silicon gasket 4 is disposed on the atomizing core jacket 3 in a sealed and sleeved manner; the atomizing core base 5 is provided with an outer screw thread 52, the atomizing core base 5 is mounted inside the oil cup 1 in a threaded manner, so as to enable the inner wall of the oil cup 1, an outer wall of the steel tube 2, the upper surface of the upper end of the atomizing core, and the atomizing core base 5 to form a sealed oil storage chamber; the atomizer machine body 10 is provided with an inner support 9, and a lower end of the oil cup 1 is provided with an outer screw thread 11. Referring to FIG. 3, when the oil cup 1 is connected to an atomizer machine body in a threaded manner, an electrode 8 at a bottom of the atomizing core is pressed by a battery inside the atomizer machine body 10 to enable an atomizing core compression spring 6 to slide upward, so that the three oil holes 32 disposed above the atomizing core jacket 3 slide through the silicon gasket 4, and the three oil holes 32 are located inside the oil storage chamber to enable the atomized liquid inside the oil storage chamber to flow to the fiber rope to be heated and evaporated by the electrical heating wire 71; and referring to FIG. 2, when the oil cup 1 is unscrewed or the oil cup 1 is screwed off, the atomizing core slides downward under an effect of a returning force of the spring 6, so that the three oil holes 32 disposed above the atomizing core jacket 3 slide through the silicon gasket 4, and the three oil holes 32 are located below the silicon gasket 4, so as to seal the oil storage chamber, thereby effectively preventing the atomized liquid from being seeped.

In an anti-seepage atomizer of an embodiment, on the basis of the preceding technical solution, specifically, the atomizing structure may be an electric induction heating structure. Alternatively, the atomizing structure may be an ultrasonic atomizing structure or the like.

In an anti-seepage atomizer of an embodiment, on the basis of the preceding technical solution, specifically, the oil cup, the atomizing core, or the atomizing core base is provided with an identification code structure, and the atomizer machine body is provided with a code reading structure corresponding to the identification code structure. If a bottom of the atomizing core base is provided with a conductive contact point arranged according to a specific structure, and the atomizer machine body is provided with a conductive contact point array corresponding to the conductive contact point, when the oil cup is inserted into a matching atomizer machine body, a correct contact point in the conductive contact point array of the atomizing core base is switched on, so that the atomizing core can work and otherwise, the atomizing core cannot be powered on or work, thereby ensuring that the atomizing core and the atomizer machine body are used in a manner of matching each other. Similarly, the oil cup, the atomizing core, or the atomizing core base may also be provided with a radio frequency tag, a two-dimensional code, an NFC tag, an identification chip, or the like, and a corresponding reading module is disposed at a corresponding position of the atomizer machine body, which can also ensure that the atomizing core and the atomizer machine body are used in a manner of matching each other.

In an anti-seepage atomizer of an embodiment, on the basis of the preceding technical solution, specifically, a control main board is disposed inside the atomizer machine body, and a surface of the atomizer machine body is further provided with a display screen electrically connected to the control main board. A working state of the atomizer can be directly viewed by using the display screen.

In an anti-seepage atomizer of an embodiment, on the basis of the preceding technical solution, specifically, a wireless transmit module electrically connected to the control main board is further disposed inside the atomizer. The wireless transmit module may be used to connect to an intelligent terminal such as a mobile phone, so as to collect data and learn a use state of the atomizer in real time.

The above describe a heating and atomizing apparatus of the present invention and are used to help understand the present invention. However, implementation manners of the present invention are not limited by the foregoing embodiments, and all of equivalent replacement manners, such as the variation, modification, substitution, combination, and simplification, that are made without departing from the principle of the present invention fall within the protection scope of the present invention.

## Claims

1. An anti-seepage atomizer, comprising an oil cup and a hollow atomizing core, wherein a hollow tube is disposed at a center of the oil cup in a manner of extending inward and downward from a top, an upper end of the atomizing core is movably inserted into the hollow tube in a sealed manner from a place below the hollow tube, a lower end of the atomizing core is movably disposed inside an atomizing core base in a sleeved manner, an inner wall of the oil cup, the hollow tube, an upper surface of the upper end of the atomizing core, and an upper surface of an atomizing core base form a sealed oil storage chamber, the middle of the atomizing core is provided with at least one oil hole in communication with an atomizing structure inside the atomizing core, if the atomizing core slides downward, the oil hole is hidden inside the atomizing core base so as to isolate the oil storage chamber from the atomizing structure in a sealed manner, and if the atomizing core slides upward, the oil hole is located inside the oil storage chamber so as to make the oil storage chamber in communication with the atomizing structure.

2. The anti-seepage atomizer according to claim 1, wherein an elastic structure is disposed between the atomizing core and the atomizing core base, the atomizing core slides upward to elastically deform the elastic structure, and the atomizing core slides downward to elastically recover the elastic structure.

3. The anti-seepage atomizer according to claim 1 or 2, wherein: the atomizing structure is a spiral heating body, a long strip-shaped heating body, a sheet-shaped heating body, or a porous circular tube-shaped heating body that is disposed in a same direction as that of the hollow tube, and the spiral heating body, the long strip-shaped heating body, the sheet-shaped heating body, or the porous circular tube-shaped heating body is wrapped by an absorption fiber around.

4. The anti-seepage atomizer according to claim 3, wherein the spiral heating body, the long strip-shaped heating body, the sheet-shaped heating body, or the porous circular tube-shaped heating body performs resistance heating or electromagnetic heating.

5. The anti-seepage atomizer according to claim 1 or 2, wherein the atomizing structure is a hollow heating body, the atomized liquid is guided to a hollow body, the hollow heating body is provided with a micro hole, steam-fog generated by means of atomization when the hollow heating body performs heating is discharged through the micro hole, and the hollow heating body performs resistance heating or electromagnetic heating.

6. The anti-seepage atomizer according to claim 1 or 2, wherein the atomizing structure is a fiber rope disposed inside an atomizing core cavity, and a surface of the fiber rope is wound by an electrical heating wire.

7. The anti-seepage atomizer according to claim 1 or 2, further comprising an atomizer machine body, wherein a pressing structure is disposed inside the atomizer machine body, and when the oil cup is connected to the atomizer machine body, the pressing structure presses the atomizing core to enable the atomizing core to slide upward.

8. The anti-seepage atomizer according to claim 7, wherein the oil cup, the atomizing core, or the atomizing core base is provided with an identification code structure, and the atomizer machine body is provided with a code reading structure.

9. The anti-seepage atomizer according to claim 8, wherein the identification code structure is a conductive contact point, a radio frequency tag, a two-dimensional code, a graphical code, or an identification chip that is arranged according to a specific structure.

10. The anti-seepage atomizer according to claim 7, wherein a control main board is disposed inside the atomizer machine body, a surface of the atomizer machine body is further provided with a display screen electrically connected to the control main board, and a wireless transmit module electrically connected to the control main board is further disposed inside the atomizer machine body.
